Europäisches Patentamt

**European Patent Office**

Office européen des brevets

(11) Publication number:

**0 156 592**

**A2**

(12) **EUROPEAN PATENT APPLICATION**

(21) Application number: **85301756.4**

(51) Int. Cl.⁴: **A 61 K 9/48**

(22) Date of filing: **14.03.85**

(30) Priority: **21.03.84 US 591738**

(43) Date of publication of application:
**02.10.85 Bulletin 85/40**

(84) Designated Contracting States:
**AT BE CH DE FR IT LI LU NL SE**

(71) Applicant: **AMERICAN HOME PRODUCTS
CORPORATION
685, Third Avenue
New York, New York 10017(US)**

(72) Inventor: **Kalidindi, Sanyasi Raju
36B Arlington Square Apartments 1149 Mulbery Lane
Greenville North Carolina(US)**

(72) Inventor: **Giannini, Robert Peter
508 Meadowbrook Road East Norriton
Montgomery Pennsylvania(US)**

(72) Inventor: **Gaskill, James Lawrence
Apartment M-307 Sugartown and Avon Roads
Devon Pennsylvania(US)**

(72) Inventor: **Russo, Emanuel Joseph
8 Morris Circle Wayne
Delaware Pennsylvania(US)**

(74) Representative: **Wileman, David Francis et al,
c/o John Wyeth and Brother Limited Huntercombe Lane
South
Taplow Maidenhead Berkshire SL6 OPH(GB)**

(54) Substained release pharmaceutical capsules.

(57) The invention concerns gastrointestinal, pH-independent, sustained-release pharmaceutical unit dosage form comprising a non-compressed mixture of a therapeutic agent and from about 10 to about 60 per cent by weight of a high molecular weight hydroxypropylmethylcellulose, and a method for production thereof in hard gelatin capsules.

EP 0 156 592 A2

This invention relates to sustained release pharmaceutical capsules, more particularly to such capsules containing a high molecular weight hydroxypropylmethylcellulose.

The convenience and advantages of administering a single dose of medication which provides prolonged or sustained-release as opposed to the administration of a number of single doses at regular intervals are indisputable. Conventionally, sustained release is achieved by controlling dissolution and/or diffusion of the medicament from the dosage form. Several carrier materials which are employed for this purpose include waxes, fatty materials, polymers, natural, synthetic and semi-synthetic gums, etc. Among the gums, hydroxypropylmethylcelluloses constitute an important class of materials because of their pH-independent effect as well as their synthetic origin as opposed to the natural gums such as alginates, Karaya, Guar, Locust bean, etc.

The use of hydroxypropylmethylcelluloses as carrier bases in sustained action tablets has been well-established. For example, Christensen et al. (U.S. Patent 3,065,143) teach sustained-release tablets which contain a hydroxypropylmethylcellulose which, upon contact with gastric fluid, swells and forms a water-impermeable barrier on the tablet surface thereby providing sustained release by diffusion of drug through the barrier. It is clear from their teachings that the gum has to be pressed into a tablet for it to work as a sustained-action agent. Sheth et al (U.S. Patent 4,167,558) disclose sustained-release tablets containing hydroxypropylmethylcellulose 60 HG. Again the dosage form is tablet and in addition there is a restriction on the density of the tablets to assure buoyancy and release the whole of the medicament in the stomach. Lowey et al. (U.S. Patent 3,870,790) disclose a solid, compressed buccal product containing low molecular weight hydroxypropylmethylcellulose which has been modified by humidification and air-drying. Similarly, Schor (U.S. Patent 4,226,849) disclosed the invention of tablets, lozenges, suppositories and/or other compressed dosage forms, which have prolonged-release wherein the hydroxypropylmethylcellulose has been subjected to hydrolysis and oxidation to generate a desired minimum concentration of carbonyl and carboxyl groups. The hydroxypropylmethylcellulose used in these teachings are of low molecular weight. In another patent (U.S. Patent 4,389,393) Schor et al. disclose the use of high molecular weight hydroxypropylmethylcelluloses in low concentrations for achieving sustained drug release action from compressed solid dosage forms. The hydroxypropylmethylcelluloses used by Schor et al. have a molecular weight above 50,000, a methoxyl content of 16-24% and hydroxypropoxyl content of 4-32%. Thus, Schor et al. (4,389,393) require that the material be compressed and it is clear from these teachings and those of Christensen et al. (U.S. Patent 3,065,143), Sheth et al. (4,167,558), Lowey et al. (3,870,790) and Schor et al. (4,226,849 and 4,389,393) that compression is essential for the formation of a diffusion barrier layer on the surface of the oral dosage form to provide sustained action.

Sheth and Tossounian (U.S. Patent 4,126,672) showed that hydroxypropylmethylcellulose provides sustained release in a buoyant capsule dosage form. However, because of the buoyancy constraints, the density of the capsules may have to be adjusted by the use of a fatty material so that the capsules float in the gastric fluid. It is clear from their teachings that floating is essential for their concept to work. Henderson et al (U.S. Patent 3,427,378) disclose capsules for sustained-release of medicament wherein a gum such as sodium alginate is incorporated into the pharmaceutical formulation in an amount of from 70 to 99 per cent by weight and preferably 85 to 98 per cent and the capsules are, as a critical feature, completely filled.

- 4 -

This invention provides a unit dosage form for administration of a therapeutic agent, which does not require expensive compression and granulation processing characteristic of the prior art sustained-release tablets, or density control restraints characteristic of the prior art capsules, while providing substantially the same slow drug release heretofore obtained with compressed tablets. The unit dosage form of this invention provides sustained release of drug independent of gastrointestinal pH and is therefore equally effective both in the stomach and intestine. By "independent", it is not meant that no change in dissolution rate occurs with change of pH, but that any change is so insubstantial that drug delivery is not meaningfully altered.

In essence, it has been discovered that pharmaceutical compositions formulated with relatively high molecular weight hydroxypropylmethylcellulose (>50,000) which heretofore have been employed in the production of compressed unit dosage forms such as tablets, buccal lozenges, suppositories, etc. may be used without compression in hard gelatin capsules without loss of the sustained release effect observed in compressed tablets. Although the pharmaceutical literature has well documented the necessity for compressing hydroxypropylmethylcellulose-containing drug formulations to provide an external surface barrier to drug release, or for control of the density of an encapsulated drug formulation compounded with hydroxypropylmethylcellulose to provide buoyancy and hold the capsule in the stomach, applicants have found that neither of these preparative restrictions are necessary to provide a sustained release unit dosage capsule for administration. The formulations employed in this invention are not tailored to ensure buoyancy in gastric fluids and they are equally effective when administered in such manner as to be retained with a food bolus passing into the small intestine. No special attention or limitation as to internal porosity is required with the formulations of this invention.

Thus, in accordance with this invention, there is provided a gastro-intestinal, pH-independent, sustained-release pharmaceutical unit dosage form comprising a hard gelatin capsule containing a pulverulent admixture of a therapeutically active medicament; from about 10 to about 60 per cent by weight of at least one hydroxypropylmethylcellulose possessing a methoxy content of 16 to 24 weight per cent, a hydroxypropoxyl content of 4 to 32 weight per cent and a number average molecular weight of at least 50,000; and a pharmaceutically acceptable, nonpharmacologically functional adjuvant therefor.

The hydroxypropylmethylcelluloses employed in production of the sustained-release capsules are commercially available. In essence, any hydroxypropylmethylcellulose (HPMC) possessing the characteristics of Methocel® K4M, K15M and K100M are applicable, individually and as mixtures. Each of these hydrophilic materials have a methoxy content of about 16 to about 24 weight per cent, a hydroxypropyl content of about 4 to about 32 weight per cent and a number average molecular weight of at least 50,000. The proportion of hydroxypropylmethyl-cellulose to total capsule weight may vary from about 10 to about 60 per cent to provide release rates of drug over a 6 to about 12 hour period. Although the release rates achieved with hard gelatin capsules containing drug formulations and hydroxypropylmethylcellulose in accordance with this invention will differ somewhat depending upon the specific drug being compounded, the sustained release effect is readily achieved. Modification of the release rate may be attained by altering the hydroxypropylmethylcellulose to drug ratio within the limits indicated above. Generally, an increased content of hydroxypropylmethylcellulose will prolong the release rate of a given drug as may be seen from the experimental data presented, infra.

An optimum release rate is obtained when near total dissolution of the therapeutic agent at a substantially uniform rate is achieved over a period of from six to twelve hours and preferably in about eight hours.

Any therapeutic agent suitable for oral administration may be formulated in conformity with this disclosure and encapsulated to provide a sustained release unit dosage capsule. Thus, tranquillisers, analgesics, antihypertensives and similar drugs may be readily formulated for use in sustained release capsule form.

In addition to the therapeutic agent and hydroxypropylmethylcellulose, other conventional additives such as fillers and binders (microcrystalline cellulose, lactose, dicalcium phosphate dihydrate, etc.); lubricants (magnesium stearate, stearic acid, etc.); glidants (colloidal silicon dioxide, talc, etc.); hydrophilic gums (methylcellulose, hydroxypropylcellulose, hydroxyethylcellulose, sodium carboxymethylcellulose, etc.); disintegrants (starch, sodium starch glycolate, etc.); preservatives (methylparahydroxy benzoate, benzoic acid, etc.); antioxidants (ascorbic acid, sodium bisulfite etc.) and colourants (certified dyes) may be employed in the formulations for filling hard gelatin capsules without materially changing the sustained release rate of medicament achieved with the indicated high molecular weight hydroxypropylmethylcellulose material.

The pharmaceutical compositions of this invention are prepared by blending an admixture of the ingredients and filling hard gelatin capsules therewith by hand or machine. Where the particular size of any one component of the composition, or the mixture in its entirety, is of such size as to detract from the production of a homogeneous or near homogeneous blend for subsequent processing, or the desired performance of the finished dosage form (e.g. dissolution, stability, uniformity, weight variation, etc.), that component or the entire mixture may be milled to any desired size prior to a final blending and capsule filling.

Accordingly this invention also provides a method for producing a gastrointestinal pH independent sustained release pharmaceutical unit dosage form comprising a hard gelatin capsule containing a pulverulent admixture of a therapeutically active medicament; from about 10 to about 60 per cent by weight of at least one hydroxypropylmethylcellulose possessing a methoxy content of 16 to 24 weight per cent, a hydroxy-propoxyl content of 4 to 32 weight per cent and a number average molecular weight of at least 50,000; and a pharmaceutically acceptable, nonpharmacologically functional adjuvant therefor, which comprises blending the above-stated ingredients and filling them into said hard gelatin capsules.

The following examples illustrate several specific formulations for filling hard gelatin capsules to produce sustained release doses of the indicated therapeutic agents. For purposes of this illustration, the drugs used were:

| | |
|---|---|
| Oxazepam | 7-chloro-1,3-dihydro-3-hydroxy-5-phenyl-2H-1,4-benzodiazepin-2-one; |
| Ciramadol | 1-cis-2-($\alpha$-dimethylamino-m-hydroxybenzyl)-cyclohexanol; |
| Guanabenz | (E)-[(2,6-dichlorobenzylidene)amino]-guanidine; and |
| Lorazepam | 7-chloro-5-(o-chlorophenyl)-1,3-dihydro-3-hydroxy-2H-1,4-benzodiazepin-2-one. |

- 8 -

### Example 1

#### Hydroxypropylmethylcellulose 10 per cent

|  | mg |
|---|---|
| Oxazepam USP | 30.00 |
| Hydroxypropylmethylcellulose 2208 USP | 37.50 |
| Microcrystalline Cellulose NF | 94.00 |
| Lactose USP Hydrous | 206.75 |
| Colloidal Silicon Dioxide NF | 3.75 |
| Magnesium Stearate NF | 3.00 |
| TOTAL | 375.00 |

### Example 2

#### Hydroxypropylmethylcellulose 20 per cent

|  | mg |
|---|---|
| Oxazepam USP | 30.00 |
| Hydroxypropylmethylcellulose 2208 USP | 75.00 |
| Microcrystalline Cellulose NF | 94.00 |
| Lactose USP Hydrous | 169.25 |
| Colloidal Silicon Dioxide NF | 3.75 |
| Magnesium Stearate NF | 3.00 |
| TOTAL | 375.00 |

### Example 3

#### Hydroxypropylmethylcellulose 30 per cent

|  | mg |
|---|---|
| Oxazepam USP | 30.00 |
| Hydroxypropylmethylcellulose 2208 USP | 112.00 |
| Microcrystalline Cellulose NF | 94.00 |
| Lactose USP Hydrous | 131.75 |
| Colloidal Silicon Dioxide NF | 3.75 |
| Magnesium Stearate NF | 3.00 |
| TOTAL | 374.50 |

- 9 -

## Example 4

### Hydroxypropylmethylcellulose 25 per cent

|  | mg |
|---|---|
| Ciramadol Hydrochloride | 68.80 |
| Hydroxypropylmethylcellulose 2208 USP | 112.50 |
| Microcrystalline Cellulose NF | 110.26 |
| Lactose USP Hydrous | 152.27 |
| Colloidal Silicon Dioxide NF | 4.50 |
| Magnesium Stearate NF | 1.67 |
| TOTAL | 450.00 |

## Example 5

### Hydroxypropylmethylcellulose 60 per cent

|  | mg |
|---|---|
| Guanabenz Acetate | 20.20 |
| Hydroxypropylmethylcellulose 2208 USP | 90.00 |
| Lactose USP Hydrous | 39.05 |
| Magnesium Stearate NF | 0.75 |
| TOTAL | 150.00 |

## Example 6

### Hydroxypropylmethylcellulose 40 per cent

|  | mg |
|---|---|
| Lorazepam | 2.00 |
| Hydroxypropylmethylcellulose 2208 USP | 40.00 |
| Microcrystalline Cellulose NF | 30.00 |
| Lactose USP Hydrous | 27.50 |
| Magnesium Stearate NF | 0.50 |
| TOTAL | 100.00 |

In each of the preceding examples, all the ingredients were mixed together in a Twin-shell® blender for 10 minutes, milled through a Fitz Mill®, and mixed again for 10 minutes in the same blender. The blend was then filled into hard gelatin capsules using a Zanasi LZ64 capsule-filling machine.

The above formulations provide sustained-release for 6-12 hours in vitro as shown in Table 1. The dissolution rate study employed to obtain the data reported in Table 1 was performed in an apparatus conforming to that described as Apparatus 2 in U.S. Pharmacopeia XX page 959 (1980) by the method therein described, at 50 r.p.m. or 100 r.p.m. The dissolution medium was 500 ml 0.1 N HCl for the first hour after which 500 ml phosphate buffer was added to afford a pH of 7.4, comparable to that found in the intestine. A spring coil was employed to sink the capsules and ensure that they did not float.

Table I:    Dissolution Data for Various Sustained-Action Capsules Containing Hydroxypropylmethyl cellulose Carrier Base.
(% Drug Dissolved as an Average of Six Capsules)

| Formulation of Example No. | 1 Hour | 2 Hours | 4 Hours | 6 Hours | 8 Hours | 12 Hours |
|---|---|---|---|---|---|---|
| 1 | 20 | 57 | 90 | 97 | - | - |
| 2 | 26 | 56 | 79 | 86 | 88 | 94 |
| 3 | 15 | 36 | 55 | 69 | 79 | 92 |
| 4 | 37 | 60 | 82 | 95 | 100 | - |
| 5 | 28 | 47 | 68 | 84 | 93 | 94 |
| 6 | 20 | 32 | 50 | 67 | 82 | 94 |

To illustrate the comparable dissolution rates between compressed tablets and the capsules of this invention employing the same formulation, the following data was obtained with the appropriate formulations disclosed, supra, and with that of Example 7.

- 11 -

## Example 7

Hydroxypropylmethylcellulose 38.6 per cent

|  | mg |
|---|---|
| Guanabenz Acetate | 20.2 |
| Hydroxypropylmethylcellulose 2208, USP | 61.7 |
| Magnesium stearate, NF | 0.55 |
| Microcrystalline cellulose, NF | 22.0 |
| Lactose, USP | 45.6 |
| Sodium starch glycolate, NF | 10.0 |
| **TOTAL** | 160.0 |

The tablets were conventionally prepared and the capsules were prepared by machine-filling as described, supra. The procedure followed in obtaining this dissolution rate data was the same as that disclosed, supra, except that the study employing guanabenz were run in all acid (0.1N HCl) without addition of buffer, and all samples were run at a paddle speed of 50 RPM.

Table II: Dissolution Data Comparing Tablets and Capsules Prepared with the same powder blend or granulation. (% Dissolved as an Average of Six Capsules and Tablets)

| Time (Hours) | Oxazepam with 20% HPMC 2208 USP | | Guanabenz with 38.6% HPMC 2208 USP | | Ciramadol with 25% HPMC 2208 USP | |
|---|---|---|---|---|---|---|
| | Tablets | Capsules | Tablets | Capsules | Tablets | Capsules |
| 1 | 12.5 | 13.5 | 35.0 | 37.5 | 26.5 | 37 |
| 2 | 22.5 | 22.5 | 52.0 | 56.0 | 41.5 | 60 |
| 4 | 33.0 | 32.0 | 77.0 | 80.0 | 57.5 | 82 |
| 6 | 40.0 | 39.0 | 93.0 | 92.0 | 75.0 | 95 |
| 8 | 46.0 | 46.0 | 93.0 | 98.0 | 85.0 | complete |
| 10 | 52.5 | 52.0 | 95.5 | 98.0 | 93.5 | complete |
| 12 | 56.5 | 55.5 | 97.0 | 96.0 | 96.5 | complete |

- 12 -

To demonstrate that there is no property imparted to the capsules of this invention by the encapsulating machine, the data presented in Table III compares the dissolution rate of hand-filled capsules with those containing the same formulations when filled with a Zanasi LZ64 encapsulating machine.  The formulations employed were those shown in Examples 2 and 6.

Table III:    Dissolution of Hand-Filled and Machine-Filled Capsules
              (% Dissolved as an Average of Six Capsules)

| Time (Hours) | Oxazepam (Example 2) | | Lorazepam (Example 6) | |
| --- | --- | --- | --- | --- |
| | Hand filed | Machine filled | Hand filled | Machine filled |
| 1 | 17 | 26 | 24 | 20 |
| 2 | 44 | 56 | 37 | 32 |
| 4 | 68 | 79 | 55 | 50 |
| 6 | 80 | 86 | 71 | 67 |
| 8 | 81 | 88 | 88 | 82 |
| 12 | 84 | 94 | 99 | 94 |
| 20 | 91 | 98 | 100 | 94 |

From these data, the preferred quantity of defined hydroxypropylmethylcellulose for general incorporation into pharmaceutical formulations for encapsulation can be seen to be from about 10 to about 50 weight per cent.  For the individual drugs herein exemplified the preferred quantity of defined hydroxypropylmethylcellulose is from about 15 to about 40 weight per cent with oxazepam; about 20 to about 50 weight per cent with lorazepam and from about 15 to about 50 weight per cent with ciramadol.

Thus, the advantages of capsule dosage forms with no loss of sustained drug-release presently found in compressed tablets, clearly characterises the benefits of this invention.

CLAIMS

1. A gastrointestinal pH independent sustained release pharmaceutical unit dosage form comprising a hard gelatin capsule containing a pulverulent admixture of a therapeutically active medicament; from about 10 to about 60 per cent by weight of at least one hydroxypropylmethylcellulose possessing a methoxy content of 16 to 24 weight per cent, a hydroxypropoxyl content of 4 - 32 weight per cent and a number average molecular weight of at least 50,000; and a pharmaceutically acceptable, nonpharmacologically functional adjuvant therefor.

2. A pharmaceutical unit dosage form as claimed in Claim 1 in which the hydroxypropylmethylcellulose is present in an amount of from about 10 to about 50 per cent by weight.

3. A pharmaceutical unit dosage form as claimed in Claim 1 in which the medicament is oxazepam.

4. A pharmaceutical unit dosage form as claimed in Claim 3 in which the said hydroxypropylmethylcellulose represents from about 15 to about 40 per cent of the contents of said capsule.

5. A pharmaceutical unit dosage form as claimed in Claim 1 in which the medicament is lorazepam.

6. A pharmaceutical unit dosage form as claimed in Claim 5 in which the hydroxypropylmethylcellulose represents from about 20 to about 50 per cent of the contents of said capsule.

7.    A pharmaceutical unit dosage form as claimed in
      Claim 1 in which the medicament is ciramadol.

8.    A pharmaceutical unit dosage form as claimed in
      Claim 7 in which the said hydroxypropylmethylcellulose represents between about 15 to about
      50 per cent of the contents of said capsule.

9.    A pharmaceutical unit dosage form as claimed
      in Claim 1 in which the medicament is guanabenz.

10.   A method for producing a gastrointestinal pH
      independent sustained release pharmaceutical unit
      dosage form comprising a hard gelatin capsule
      containing a pulverulent admixture of a
      therapeutically active medicament; from about 10
      to about 60 per cent by weight of at least one
      hydroxypropylmethylcellulose possessing a methoxy
      content of 16 to 24 weight per cent, a hydroxy-
      propoxyl content of 4 to 32 weight per cent and a
      number average molecular weight of at least 50,000;
      and a pharmaceutically acceptable, nonpharmacologically functional adjuvant therefor, which comprises
      blending the above-stated ingredients and filling
      them into said hard gelatin capsule.

CLAIMS FOR AUSTRIA

1.  A method for producing a gastrointestinal pH
    independent sustained release pharmaceutical
    unit dosage form comprising a hard gelatin capsule
    containing a pulverent admixture of a therapeutically
    active medicament, from about 10 to about 60 per
    cent by weight of at least one hydroxypropylmethylcellulose possessing a methoxy content of 16 to 24
    weight per cent, a hydroxypropoxyl content of
    4 to 32 weight per cent and a number average
    molecular weight of at least 50,000 and a
    pharmaceutically acceptable nonpharmacologically
    functional adjuvant therefor, which comprises
    blending the above-stated ingredients and  filling
    them into said hard gelatin capsule.

2.  A method as claimed in Claim 1 in which the
    hydroxypropylmethylcellulose is present in an
    amount of from about 10 to about 50 per cent by
    weight.

3.  A method as claimed in Claim 1 in which the medicament
    is oxazepam.

4.  A method as claimed in Claim 3 in which the hydroxypropylmethylcellulose represents from about 15 to
    about 40 per cent of the contents of the capsule.

5.  A method as claimed in Claim 1 in which the
    medicament is lorazepam.

6.  A method as claimed in Claim 5 in which the
    hydroxypropylmethylcellulose represents from about
    20 to about 50 per cent of the contents of the
    capsule.

7.  A method as claimed in Claim 1 in which the medicament is ciramadol.

8.  A method as claimed in Claim 7 in which the hydroxypropylmethylcellulose represents from about 15 to about 50 per cent of the contents of the capsule.

9.  A method as claimed in Claim 1 in which the medicament is guanabenz.